# EUROPEAN PATENT APPLICATION

(11) **EP 2 799 433 A2**
(43) Date of publication of application: **05.11.2014**
(21) Application number: 14001508.2
(22) Date of filing: 29.04.2014
(51) Int. Cl.: C07D 317/20, C07D 319/06, C07D 407/12, C10L 1/02

(54) **Acetals esters produced from purified glycerin for use and application as emollients, lubricants, plasticizers, solvents, coalescents, humectant, polymerization monomers, additives to biofuels**

(30) Priority: 29.04.2013 BR 102013010477
(71) Applicant: Glycerosolution Quimica, Ltda, CEP 04571-000 Sao Paulo (SP) (BR)
(72) Inventor: Borges Sato, Marcelo Eiji, CEP 05706-290 SAO Paulo (BR)
(74) Representative: Juncosa Miro, Jaime

(57) **Abstract**

The invention refers to a new group of acetal monoesters and diesters which have in its structure the ester function and cyclic ethers. These products have excellent properties as solvency, plasticity in polymers, solubility in polar and nonpolar means, spreadability, wetting, low volatility, non-toxicity and biodegradability. These properties make these products excellent candidates as solvents in formulations of pesticides, agricultural herbicides, for the paint and leather industry in domestic or industrial higiene formulations; as plasticizers for polymers such as polyvinyl chloride, nitrocellulose, cellulose, acrylics, polyvinyl vinyl and its copolymers; as lubricants in industrial formulations, textile industry; as emollients agents which donate oiliness for the cosmetic industry; as wetting agents that are commonly used, and as biodiesel additives to reduce the freezing point and to improve its combustion.

## Description

### Application field:

The present invention is related to a new group of acetal monoesters and diesters possessing in its structure the ester and cyclic ether function that give these products excellent properties such as solvency, plasticity in polymers, solubility in polar and nonpolar means, spreadability, wetting, low volatility, non-toxicity and biodegradability. These properties make these products excellent candidates as solvents in formulations of pesticides, agricultural herbicides for the paint industry, leather, in formulations of domestic or industrial hygiene; such as plasticizers for polymers as polyvinyl chloride, nitrocellulose, cellulose, acrylic, vinyl and polyvinyl acetate and its copolymers; as lubricants in industrial formulations, textile industry; as emollient agentswhich donateoiliness for the cosmetic industry; as humectant agents in general use;

### Description of the State of the Art

Esters produced from carboxylic acids and alcohols of carbonic chain containing 3 to 18 carbons are produced on an industrial scale and used in paint, pharmaceutical, cosmetic, plastic, agricultural and metallurgical industry.

Due to the wide variety of existing products, the products are selected in most cases concerning its performance, cost and availability in each region.

Products from renewable sources can have in some applications a higher added value than those from the petrochemical sources.

Formulations are made using more than one product to obtain the desired effects for each specific need, for example, the use of natural antioxidants in cosmetic formulations using fatty esters as agents whichdonate oliness to the skin. In industry, the products and formulations containing lubricity effects, spreadability, anti-corrosion protection, plasticity, adhesiveness, solvency, hydrophilicity, hydrophobicity are much required by the market.

The cosmetic industry in the production of creams, shampoos and sunscreens use fatty acid esters as octyl stearate, isopropyl palmitate, cetyl palmitate, combined with tocopherols to obtain the effect of oiliness and protection of the skin and hair.

The industry of domestic and industrial hygiene uses often the glycols as butyl glycol or its acetate, mono ethylene glycol, monoethylene glycol ether in formulations for cleaning as detergents, soaps, and multiuse cleaners.

In the agricultural industry, the use of ethylhexyl lactate, lactamides, toluol, xylol, methyl caprylate, methyl oleate, methyl linoleate, isoparaffins, triacetin, isoforons, dimethylamides and a diverse range of products with solvency power on the active agents are used in large volume. They are products with high solvency power and they have low ocular irritation, and they are therefore highly demanded by the industry.

The industrial inks generally use sodium dioctyl sulfosuccinate, alkylpolyglucosides such as humectants in its composition in order to ensure a good applicability in various physical surfaces such as wood, concrete, metals and plastics. Fatty esters, glycols and derivatives are used as coalescing agent in inks to reduce the minimum temperature of film formation mainly in regions where the temperatures are low.

In the metallurgical industry the cutting fluids are used in the production of metal parts to reduce physical friction and equipment wear. Methyl oleate, sorbitan trioleate trimethylolpropane are components in the formulas to give effect of lubricity in this segment. Oxidative stability, lubricity, spreadability are highly desired effects in various formulas that are developed.

Domestic compressors that exist in refrigerators, freezers and large industrial compressors use different types of lubricants. These products must be compatible with the refrigerant gases used in them, together with a high chemical stability, lubricity, spreadability, wetting, anticorrosivity to ensure a longer life for the equipment, since the compressor is the moto that guarantees the equipment operation. Low viscosity lubricants to reduce the energy consumption of equipment are a trend and a requirement of major compressor manufacturers today. Polyesters made from ethylhexanoic acid reacted with neopentyl, trimethylolpropane, pentaerythritol, alkyl benzenes and polyalkylene glycol are the main products currently used by the industry.

The biofuel industry more specifically the biodiesel industry use antioxidants such as hydroquinone, terc-butyl methyl phenol as antioxidants, additives to lower the freezing point of biodiesel produced mainly with saturated fatty acids as biodiesel from tallow and also to improve the combustion thereof. That is mainly made to prevent the diesel freezing in regions with low temperatures.

The market of plasticizers has introduced in recent years various product types in order to obtain materials of lower environmental impact, safer from the point of toxicological view with better and economically viable performance and mainly with the content of renewable raw materials.

The use of phthalates such as dibutyl phthalate, diisobutyl phthalate, diisooctyl phthalate and diisononyl phthalate, traditionally the products most used in the market have been restricted around the world due to toxicological aspects that still need technical proof in many cases.

Another traditional family are the epoxidized vegetable oils and their respective methyl and ethyl esters used as plasticizers and/or solvents.

New plasticizers named "phthalates-free" as the esters of sulfonic alkane acid, esters derived from biobutanol, diisononyl cyclohexane carboxylic ester (D1NCH); lactic acid esters, tributylcitrate, levulinic glycerol ester; poliadipates, hexyl stearate, succinic acid diesters, terephthalic acid diesters, acetylated castor oil has been marketed in recent years.

The patent EP 2245089 (WO2009102877) describes the use of methyl ester of epoxidized soybean and epoxidized soybean oil as plasticizer in formulations of polyvinyl chloride: The Patent US 8,053,468 describes the synthesis of a specific group of acetals from the glycerinreaction and levulinic acid and their applications, one of the most recent patents in this area that also considers the glycerin as one of the reagents.

Biosuccinic aciddiesters esterified with alcohols of chain with 4 to 9 carbons have also been made for the production of green plasticizers.

In all cases mentioned above, the search for a better product or formulation which gives maximum efficiency and the highest number of physical and/or physicochemical effects with the lowest cost, and that are sustainable from the environmental point of view has been intensified by the general industry.

The development of innovative products and applications of several classes aim at "obtaining more with less" is a need in order to ensure that the existing natural resources are now sufficient to guarantee a good quality of life for future generations.

### Description of the Invention

The task of this invention is to develop new products from glycerin and their respective use and application. In this invention products named carboxylic monoesters and carboxylic diesters of cyclic acetals have been developed, where the cyclic acetals are produced from glycerin, an organic aldehyde and a carboxylic acid.

The invention creates the acetal derivatives as described in patent PI 0603912-0 and P10703673 -6 from the same author in which the acetals may be formed *in situ,* i.e. acetals; and esters are formed at the same time. Preferably the glycerin used is the purified glycerin obtained according to patent BR 1020120015846, although the distilled glycerin or blond glycerin treated as market standard is contemplated in this invention. The usage of purified glycerin with 95 % purity has as main advantage the cost due to the fact that it does not pass through the distillation process which is onerous and with high energy consumption.

Another important aspect is the fact that the purified glycerin derived from the processes that use soybean oil to produce biodiesel or methyl esters contain active ingredients in the range of 100 to 1000 mg per kilogram as the tocopherols which are valuable as natural antioxidants. A significant part of the tocopherols that naturally exist in vegetable oils (see table below) is that the same are naturally drawn during the production process of biodiesel and soybean methyl esters due to the solvent effect of glycerin and methanol andare concentrated on the crude glycerin.

**Table: Typical Tocopherol and Tocotrienol existing in vegetable oils (mg/kg)**

| Oil | Total TOC | α | β | γ | δ | Total TRI | α | β | γ | δ |
|---|---|---|---|---|---|---|---|---|---|---|
| Castor | 478 | 28 | 29 | 111 | 310 | | | | | |
| Corn | 603 | 134 | 18 | 412 | 39 | | | | | |
| Cottonseed | 940 | 573 | 40 | 317 | 10 | | | | | |
| Olive | 100 | 93 | | 7 | | | | | | |
| Palm | 340 | 279 | | 61 | | 741 | 274 | | 398 | 69 |
| Rape | 695 | 272 | | 423 | | | | | | |
| (Canola) | | | | | | | | | | |
| Rice bran | 445 | 374 | 18 | 53 | | 585 | 236 | | 349 | |
| Soybean | 1000 | 90 | 680 | 680 | 230 | | | | | |
| Sunflower | 636 | 608 | 17 | 11 | | | | | | |
| Wheat germ | 2188 | 1179 | 398 | 493 | 118 | | | | | |

The image below depicts the chemical structures of the tocopherols that exist in vegetable oils. The soybean oil is the richest one in tocopherols; which act as natural antioxidants.

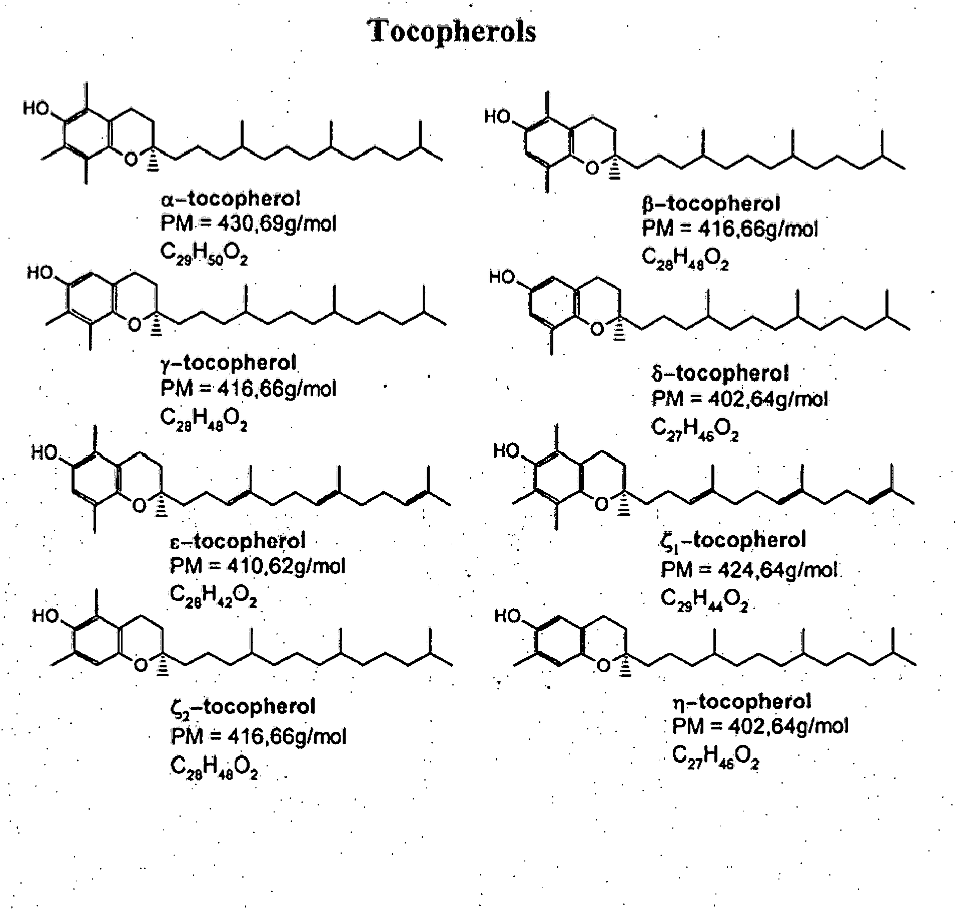

The esters are produced basically from 4 main groups of chemical intermediates:
1 - Purified glycerin 95% containing natural antioxidants or distilled glycerin or treated blondglycerin;
2 - Organic aldehydes, isobutyraldehyde, butyraldehyde 2-ethylhexaldehyde, benzaldehyde or furfuraldehyde,
3 - Monocarboxylic acids, dicarboxylic, tricarboxylic or their respective anhydrides;
   3.1 where the monocarboxylics are represented by organic acids from petrochemical origin such as acetic acid, propionic acid, butyric acid, isobutyric acid, capric acid, caprylic acid, 2 ethylhexanoic acid, isononanoic acid;
   3.1.1 or from vegetable or animal origin as the capric acid, caprylic acid, decanoic acid, lauric acid, palmitic acid, myristic acid, stearic acid, oleic acid, linoleic acid, linolenic acid, ricinoleic acid, arachidonic acid, behenic acid, lignoceric acid, and may be in pure form or more commonly found in the form of mixtures according to the source origin.
   3.2 where the dicarboxylic and tricarboxylic acids are represented by the oxalic acid, succinic acid, malonic acid, glutaric acid, adipic acid, pimelic acid, suberic acid, azelaic acid, sebacic acid, orthophthalic acid, isophthalic acid,terephthalic acid, maleic acid, fumaric acid, lactic acid, citric acid,trimellitic acid, dimerized fatty acids with 36 to 54 carbons.
   3.3 where the anhydrides are represented by the acetic anhydride, phthalic anhydride, maleic anhydride, trimellitic anhydride.
4- where the alcohol esters are the respective esters of the carboxylic, dicarboxylic and tricarboxylic acids mentioned under 3.1; 3.1.1; 3.2; 3.3, since the alcohol of the corresponding ester may be the methanol, ethanol, propanol, isobutanol, isoamyl, capric, caprylic, 2-ethylhexanol.

Several reagents are used to obtain products with different molecular sizes and chemical structures in order to better adapt to its usage, but the ester chemical function of cyclic acetal is always present in every product and claimed applications, whose basic structure is shown below: 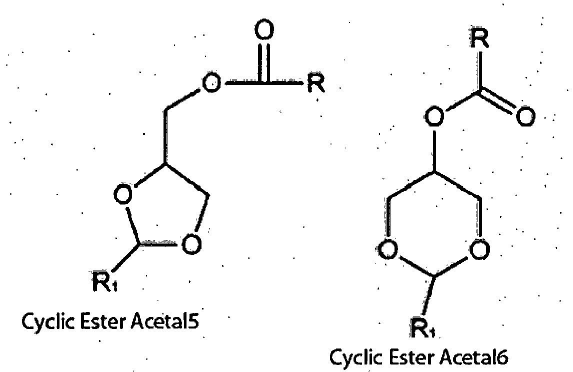 Where R1, R2 represents the portion that comes from the organic aldehyde; and R represents the portion that comes from the carboxylic acid

During the phase of formation of the cyclic acetal which functions as the reactant alcohol in the formation of products exists the formation of the acetal with heterocyclic ring composed of carbon, hydrogen, oxygen, of 5 and 6 components named in this invention cyclic acetal5 and cyclic acetal 6 which are in a weight ratio of 70/30. In order to simplify the structure of the products we show only the component cyclic acetal5 and the derivatives thereof.

The glycerin used may be the distilled glycerin usually found in the market or preferably the purified glycerin with 95% purity obtained according to the patent BR 1020120015846 that contains, in addition to its economic differential, tocopherols in a small amount that act as natural antioxidants during the applications. The technical distilled glycerintechnique, USP glycerin, treated blond glycerin may also be used in these processes.

Basic formation reaction of the acetal using organic aldehydes as reagents:

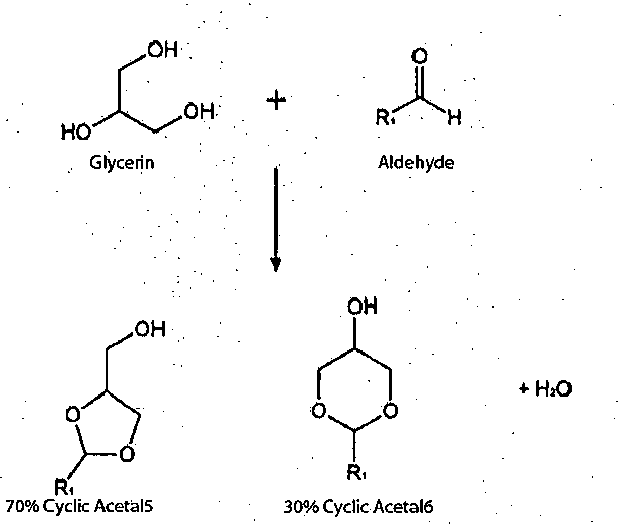

The aldehyde in the represented formula are those mentioned in item 2: isobutyraldehyde, butyraldehyde, 2-ethylhexaldehyde, benzaldehyde or furfuraldehyde.

For the synthesis of the cyclic acetal ester 3 basic techniques were used:
1 - Synthesis of acetal followed by the esterification with the carboxylic acid
2- Synthesis of acetal followed by the transesterification with carboxylic acid ester 3- Synthesis of inn situ acetal and esterification at the same time
The choice of the chemical route depends on the reactivity of the reagents, their availability and their cost in the market, but at the end the same cyclic acetal ester is always obtained regardless of the route used.

In this patent the acetal part in the molecules will be represented by the cyclic acetal6.

### Formation reaction of monoester from the acetal carboxylic acid:

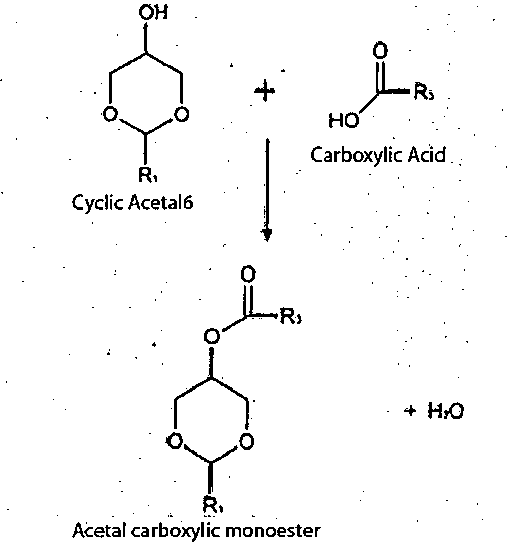

### Formation reaction of the acetal carboxilic diester:

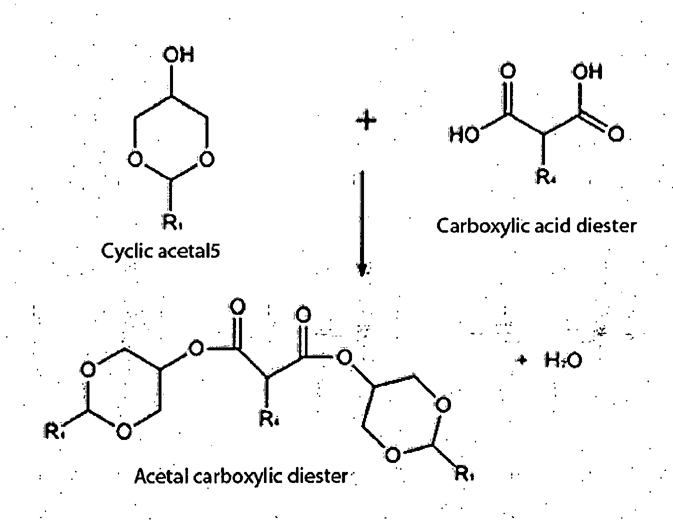

### Formation reaction of the carboxilic ester monoacetal through transesterification:

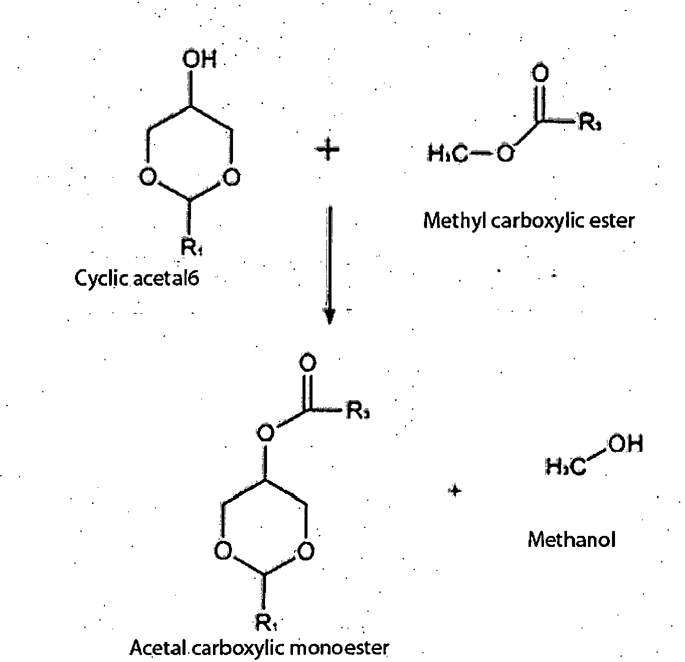

### Formation reaction of the carboxylic acid diester via transesterification:

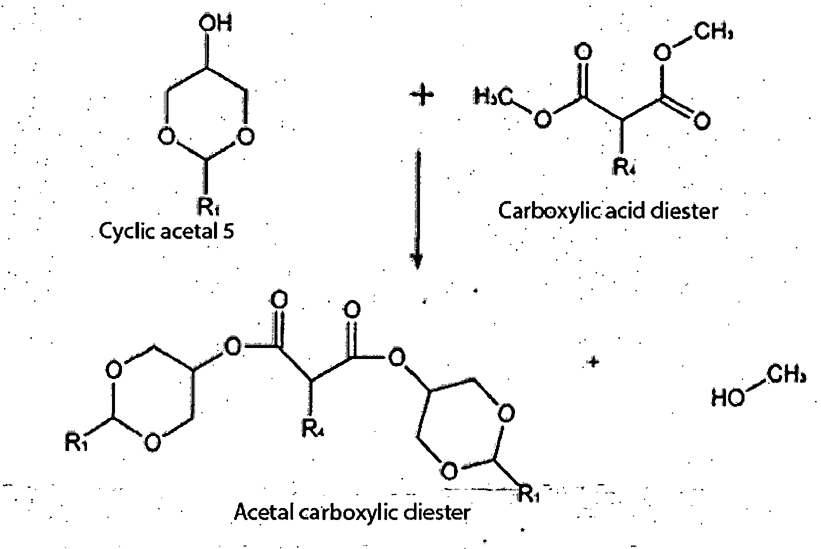

### Formation reaction of hybrid acetal diesters and alcohol via esterification:

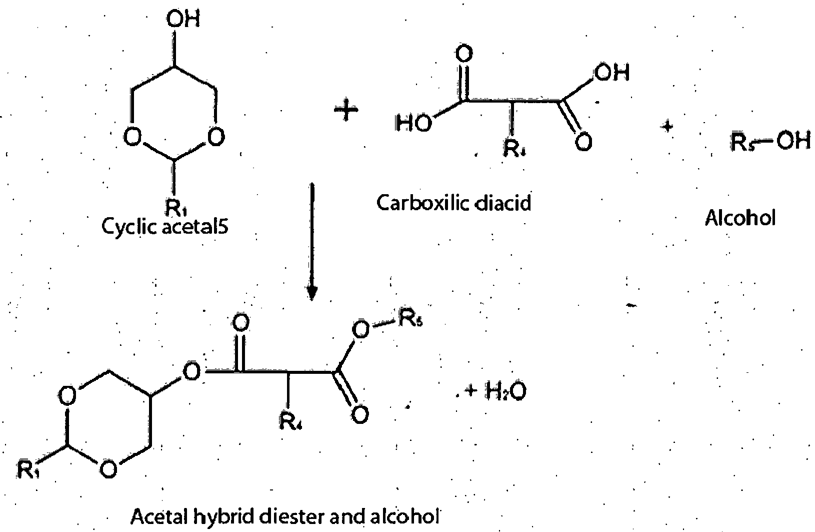

### Example 1: Synthesis of butyl laurate, isobutyl, ethylhexyl acetal esters:

In a glass reactor charge 2,105 grams of purified glycerol 95% (21.7 mol) equipped with condenser and water separator, and under continuous flow of nitrogen, add 5.0 grams of 85% phosphoric acid. Heat the reactional mass to 110 °C and add into the reaction mass 2248 (30.4 mol) grams of butyraldehyde or isobutyraldehyde or ethylhexaldehydein 60 minutes of addition. Keep reacting during 180 minutes until all the water of reaction (aprox.390 grams) is separated.

Add 3472 grams of lauric acid 99% (17.38 mol) and 5 grams of dibutyltin dilaurate. Heat 120 minutes to 180 °C and react until it reaches an index of lower acidity that 3 mgkoh/gramsof the product. Apply a vacuum of 100 mmHg to remove the excess of the reagents.
About 5700 grams of laurate of isobutyl acetal ester was obtained with 95% purity analyzed using gas chromatography coupled to a mass spectrometer (GC / MS).
The reaction yield of 95% as butyl laurate, isobutyl, ethylhexyl

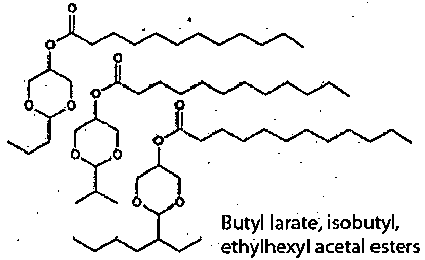

### ESTER ACETALS

### Example 2: Synthesis of butyl capric caprylate, acetal isobutyl:

In a glass reactor charge 290 grams of purified glycerol 95% equipped with condenser and water separator, and under a continuous flow of nitrogen, add 5.0 grams of 85% phosphoric acid and 250 grams of butyraldehyde or isobutyraldehyde. Heat to 120 °C and maintain it reacting during 180 minutes. Add 462 grams of caprylic capric acid and 2 grams of dibutyltin dilaurate. Heat during120 minutes to 180 °C and react until it reaches an index of acidity less than caprylate capric of. Apply
a vacuum of 100 mmHg to remove the excess of reagents.
The butyl capric caprylate, acetal isobutyl showed a purity of 97.5%.

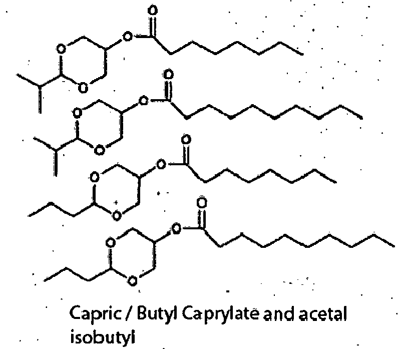

Example 3: Synthesis of capric caprylate ethylhexyl acetal: In a glass reactor charge 290 grams of purified glycerol 95% equipped with condenser and water separator, and under continuous flow of nitrogen, add 5.0 grams of phosphoric acid 85% and 500 grams of ethylhexyl aldehyde. Heat to 120°C reacting for 180 minutes. Add 462 grams of caprylic and capric acid 2 grams of dibutyltin dilaurate. Heat to 180°C for 120 minutes and react it until it reaches the acidity index less than caprylate capric of. Apply a vacuum of 100 mmHg to remove the excess of the reagents.
The capric caprylic ethylhexyl acetal showed a purity of 95.5%

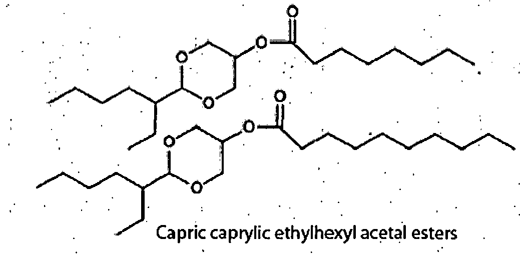

### Example 4: Synthesis of isobutyl acetal esters of lauric, myristic, palmitic, stearic fatty acid:

In a glass reactor charge 290 gramas of purified glycerol 95% equipped with condenser and water separator, and under continuos flow of nitrogen, add 2.0 grams of methane sulfonic acid 30% and 250 grams of isobutyraldehyde.
Heat to 120°C reacting for 180 minutes. Add 264 grams of palm fatty acid. Heat during 120 minutes to 160°C and react until it reaches the acidity index less than 3 mgkoh/grams of the product. Apply vacuum of 100 mmHg to remove excess of the reagents. The laurate, palmitate, myristate, isobutyl stearate acetal showed a purity of 97.5%

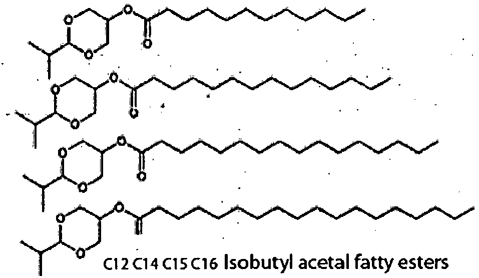

### Example 5: Synthesis of the acetal ethylhexyl esters of lauric, myristic, palmitic, stearic fatty acids.

In a glass reactor charge 290 grams of purified glycerol 95% equipped with condenser and water separator, and under continuous flow of nitrogen, add 2.0 grams of methane sulfonic acid 30% and 500 grams of etilhexiildeide. Heat to 120 °C for reacting for 180 minutes. Add 264 grams of palm fatty acid. Heat for 120 minutes to 160 ° C and react until it reaches an acidity index less than 3 mgkoh/grams. Apply vacuum of 100 mmHg to remove excess of reagents.
The laurate, palmitate, myristate, stearate, oleate, linoleate, ethylhexyl linolenate acetal showed a purity of 94.5%.

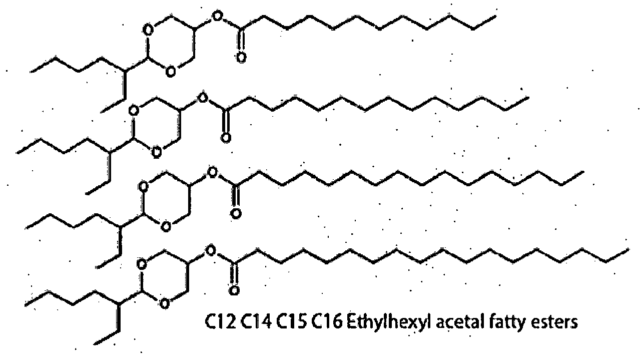

### Example 6: Synthesis of acetal butyl ester 2-ethylhexanoic acid:

In a glass reactor charge 290 grams of purified glycerol 95% equipped with condenser and water separator, and under continuous flow of nitrogen, add 2.0 grams of methane sulfonic acid 30% and 250 grams of butyraldehyde. Heat to 120°C f reacting for 180 minutes. Add 240 grams of 2-ethylhexanoic acid. Heat for 120 minutes to 160°C and react until it reaches an acidity index less than 3 mgkoh/grams of the product. Apply vacuum of 100 mmHg to remove excess of reagents.
The butyl acetal ester from 2-ethylhexanoic acid has a purity of 97%.

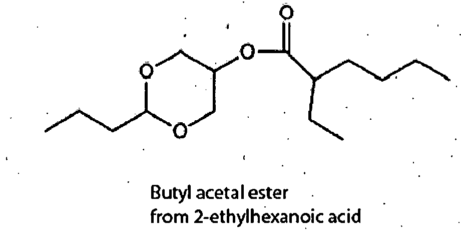

### Example 7: Synthesis of isobutyl acetal ester from 2-ethylhexanoic:

In a glass reactor charge 290 grams of purified glycerol 95% equipped with condenser and water separator, and under continuous flow of nitrogen, add 2.0 grams of methane sulfonic acid 30% and 250 grams of isobutyraldehyde.
Heat to 120 °C reacting for 180 minutes. Add 240 grams of 2-ethylhexanoic acid. Heat for 120 minutes to 160 °C and react until it reaches an acidity index less than 3 mgkoh/grams of the product. Apply vacuum of 100 mmHg to remove the excess of reagents.

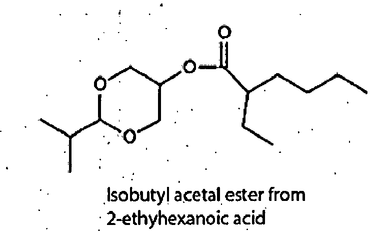

### Example 8: Synthesis of ester acetal from ethylhexyl 2-ethylhexanoic acid:

In a glass reactor charge 290 grams of purified glycerol 95% equipped with condenser and water separator, and under continuous flow of nitrogen, add 2.0 grams of methane sulfonic acid 30% and 500 grams of ethylhexyl aldehyde. Heat to 120 °C reacting or 180 minutes. Add 240 grams of 2-ethylhexanoic acid. Heat for 120 minutes to 160 °C and react until it reaches acidity index less than 3 mgkoh/grams of products. Apply vacuum of 100 mmHg to remove excess of reagents.
The acetal ethylhexyl ester from 2- ethylhexanoic acid has purity of 97%.

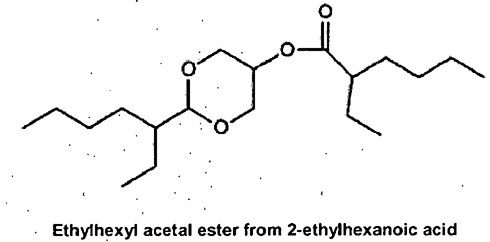

### Example 9: Synthesis of isobutyl acetal of the fatty acids from soyabean oil via esterification:

In a glass reactor charge 280 grams of purified glycerol 95% equipped with condenser and water separator, and under continuous flow of nitrogen, add 2.0 grams of methane sulfonic acid 30%%, 250 grams of isobutyraldehyde, 200 grams of fatty acid from soybean oil. Heat for 120 minutes to 160 °C and react until it reaches an acidity index lower than 3 mgkoh/grams of the product. Apply vacuum of 100 mm Hg to remove the excess of reagents.
The isobutyl acetal ester of fatty acids of soybean oil showed a purity of 98%

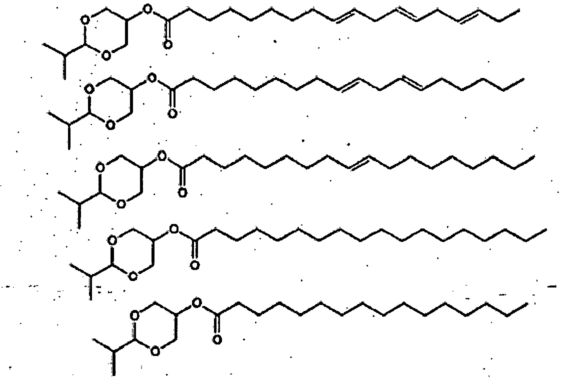

### Example 10: Synthesis of butyl acetal esters of fatty acid from soybean oil via esterification:

In a glass reactorcharge 280 grams of purified glycerol 95% equipped with condenser and water separator, and under continuous flow of nitrogen, add 2.0 grams of methane sulfonic acid 30% % 250 grams of butiraldehide, 200 grams of fatty acid from soybean oil. Heat for 120 minutes to 160 °C and react until it reaches an acidity index lower than 3 mgkoh/gram of product.
Apply vacuum of 100 mmHg to remove excess of reagents.
The butyl acetal ester of fatty acids of soybean oil showed a purity of 97%

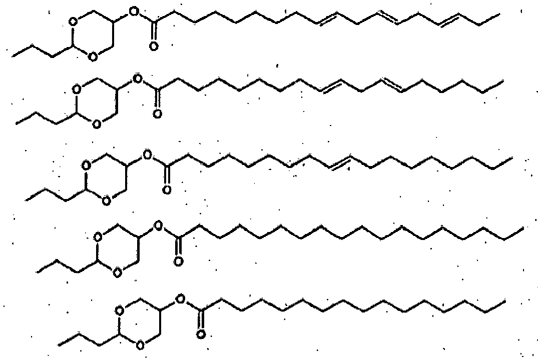

### Example 11: Synthesis of ethylhexyl acetal esters of fatty acids from soybean oil via esterification:

In a glass reactor charge 280 grams of purified glycerol 95% equipped with condenser and water separator, and under continuous flow of nitrogen, add 2.0 grams of methane sulfonic acid 30% % ethylhexyl aldehyde 500 grams, 200 grams of fatty acid from soybean oil. Heat for 120 minutes for 180°C and react until it reaches an acidity index lower than 3 mgkoh/gram of product. Apply vacuum of 100 mmHg to remove excess of reagents.
The ethylhexyl acetal ester of fatty acids from soybean oil showed a purity of 96%.

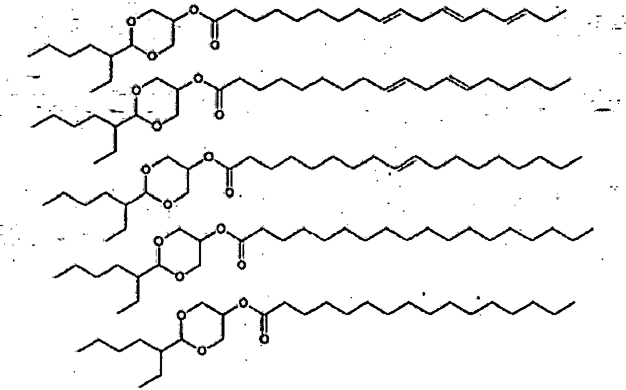

### Example 12: Synthesis of diisobutyl acetal ester from ortho phthalic anhydride.

In a glass reactor charge 2,105 grams of purified glycerol 95% equipped with condenser and water separator, and under continuous flow of nitrogen, add 6.0 grams of methane sulfonic acid, 2248 grams of isobutyraldehyde and 900 grams of ortho phthalic anhydride. Heat for 120 minutes to 180 °C and react until it reaches an acidity index lower than 3 mgkoh/gram of the product. Apply vacuum of 100 mmHg to remove excess of reagents.
The diisobutyl acetal ester from ortho phthalic anhydride showed a purity of 95%.

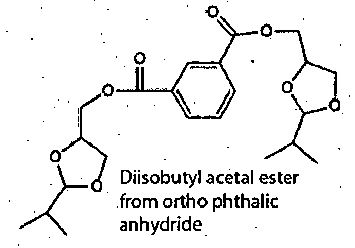

### Example 13: Synthesis of dibutyl acetal ester ortho phthalic anhydride:

In a glass reactor charge 2,105 grams of 95% equipped with condenser and water separator and under continuous flow of nitrogen, add 6.0 grams of methane sulfonic acid, 2248 grams of butiraldehyde or isobutyraldehyde and 900 grams of ortho phthalic anhydride. Heat for 120 minutes to 180 °C and react until it reaches an acidity index lower than 3 mgkoh/gram of product. Apply vacuum of 100 mmHg to remove excess of reagents.
The dibutyl and diisobutyl acetal ester ortho phthalic anhydride showed a purity above 95%.

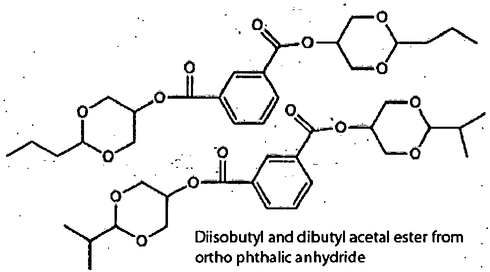

### Example 14: Synthesis of diethylhexil acetal ester of ortho phthalic anhydride:

In a glass reactor load 2,105 grams of purified glycerol 95% equipped with condenser and water separator, and under continuous flow of nitrogen, add 6.0 grams of methane sulfonic acid, 3000 grams of ethylhexyaldehyde and 900 grams of ortho phthalic anhydride. Heat for 120 minutes to 180 °C and,react until it reaches an acidity index lower than 3 mgkoh/gram of the product. Apply vacuum of 100 mmHg to remove excess of reagents.
The diethylhexyl-ester acetal from ortho phthalic anhydride showed a purity of 96%.

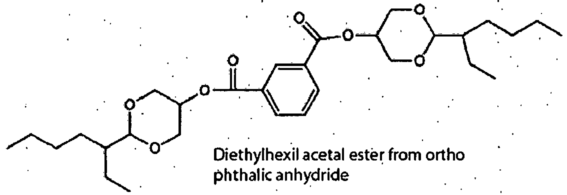

### Example 15: Synthesis of diisobutyl acetal ester from adipic acid:

In a glass reactor charge 210 grams of purified glycerol 95%, 0.65 grams of paratoluenesulfonic acid, 220 grams of butyraldehyde or isobutyraldehyde and 100 grams of adipic acid. Heat for 120 minutes to 160 °C and react until it reaches an acidity index lower than 3 mgkoh/gram of product. Apply vacuum of 100 mmHg to remove excess of reagents.
The dibutyl and diisobutyl acetal ester from adipic acid showed a purity of 98%.

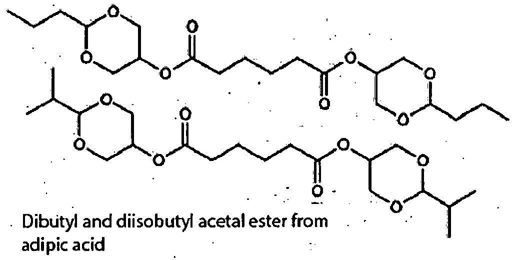

### Example 16: Synthesis of diethylhexyl acetal ester from adipic acid:

In a glass reactor charge 210 grams of purified glycerol 95%, 0.65 grams of paratoluene sulfonic acid, 400 grams of ethylhexyl aldehyde, 100 g of adipic acid. Heat for 120 minutes to 160 °C and react until it reaches an acidity index lower than 3 mgkoh/gram of product. Apply vacuum of 100 mmHg to remove excess of reagents.
The diethylhexyl acetal ester from adipic acid has a purity of 98%.

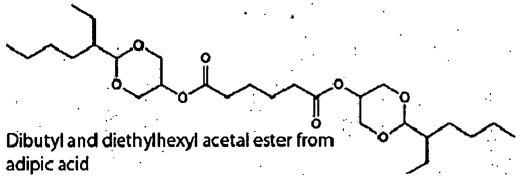

### Example 17: Synthesis of dibutyl or diisobutyl acetal ester from terephthalic acid:

In a glass reactor charge 2.11 grams of purified glycerol 95% equipped with condenser and water separator, and under continuous flow of nitrogen, add 6.0 grams of methane sulfonic acid, 220 grams of butyraldehyde or isobutyraldehyde and 93g of terephthalic acid Heat for 120 minutes to 180 °C and react until it reaches an acidity index lower than 3 mgkoh/gram of the product. Apply vacuum of 100 mmHg to remove excess of reagents.
The diisobutyl acetal ester from terephthalic acid showed a purity of 95%.

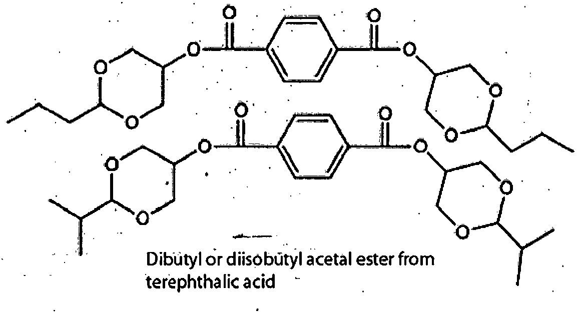

### Example 18: Synthesis of diethylhexyl acetal ester of terephthalic acid:

In a glass reactor charge 2,11 grams of purified glycerol 95% equipped with condenser and water separator, and under continuous flow of nitrogen, add 6.0 grams of methane sulfonic acid, 440 grams of ethylhexyl aldehydeand 93 grams of terephthalic acid. Heat for 120 minutes to 180 °C and react until it reaches an acidity index lower than 3 mgkoh/gram of product. Apply vacuum of 100 mmHg to remove excess of reagents. The diethylhexyl acetal ester from terephthalic acid showed a purity of 96%.

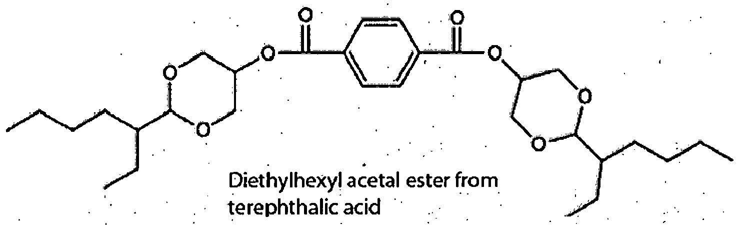

### Example 19: Synthesis of dibutyl or diisobutyl acetal ester of succinic acid:

In a glass reactor charge 220 grams of purified glycerol 95% equipped with condenser and water separator, and under continuous flow of nitrogen, add 0.7 grams of methane sulfonic acid, 230 grams of butyraldehyde or isobutyraldehyde and 80 grams of succinic acid. Heat for 120 minutes to 180 °C and react until it reaches an acidity index lower than 3 mgkoh/gram of product. Apply vacuum of 100 mmHg to remove excess of reagents.
The dibutyl or diisobutyl acetal ester from succinic acid showed a purity of 95%.

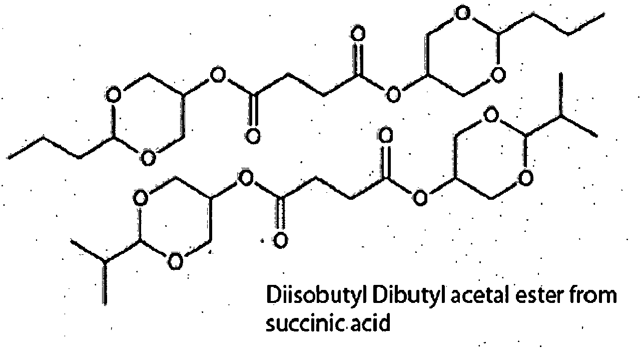

### Example 20: Synthesis of isoamyl hybrid esters; diisobutyl, butyl, ethylhexyl acetals of adipic acid:

In a glass reactor charge 220 grams of purified glycerol 95% equipped with condenser and water separator, and under continuous flow of nitrogen, add 0.7 grams of methane sulfonic acid, 230 grams of isobutyraldehyde or isobutyraldehyde (500grams in the case of ethylhexyl aldehyde, 120 grams of isoamyl alcohol and 120 grams of adipic acid). Heat for 120 minutes to 180°C and react until it reaches an acidity index lower than 3 mgkoh/gram of the product. Apply vacuum of 100 mmHg to remove excess of reagents. The isoamyl hybrid esters; isobutyl, butyl, ethylhexyl acetals of adipic acid has a purity over 90%.

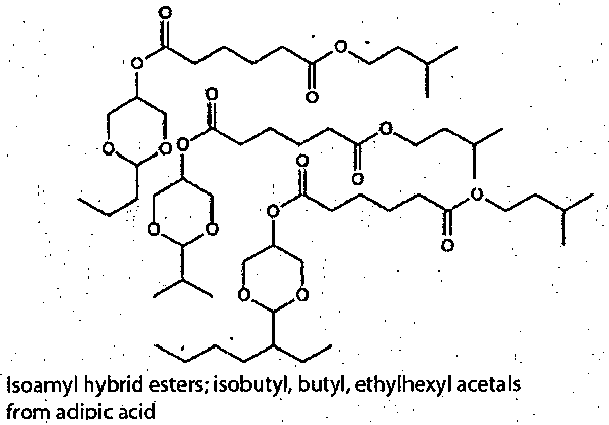

### Example 21: Synthesis of isoamyl hybrid esters; diisobutyl, butyl, ethylhexyl acetals of orthophthalic anhydride.

In a glass reactor charge 200 grams of purified glycerol 95% equipped with condenser and water separator, and under continuous flow of nitrogen; add 0.7 grams of methane sulfonic acid, 230 grams of isobutyraldehyde or isobutyl aldehyde (500grams in the case of ethylhexyl aldehyde, 120 grams of isoamyl alcohol and 120 grams of adipic acid). Heat for 120 minutes to 180 °C and react until it reaches an acidity index lower than 3 mgkoh/gram of product. Apply vacuum of 100 mmHg to remove excess of reagents.
The isoamyl hybrid esters; isobutyl, butyl, ethylhexyl acetals of phthalic anhydride has purity over 93%.

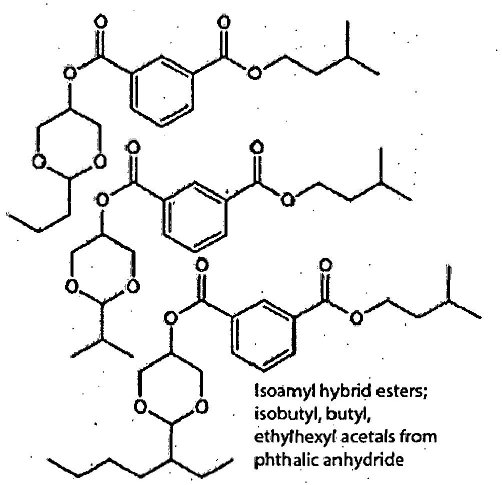

### Example 22: Synthesis of isoamyl hybrid esters; diisobutyl, butyl, ethylhexyl acetals of terephthalic acid:

In a glass reactor charge 220 grams of purified glycerol 95% glycerol equipped with condenser and water separator, and under continuous flow of nitrogen, add 0.7 grams of methane sulfonic acid, 230 grams of isobutyraldehyde or isobutyraldehyde (500grams in the case of ethylhexyl aldehyde, 120 grams of isoamyl alcohol and 120 grams of terephthalic acid). Heat for 120 minutes to 180 °C and react until it reaches an acidity index lower than 3 mgkoh/gram of product. Apply vacuum of 100 mmHg to remove excess of reagents.
The isoamyl hybrid esters; isobutyl, butyl, ethylhexyl acetals of terephthalic acid has purity over 90%.

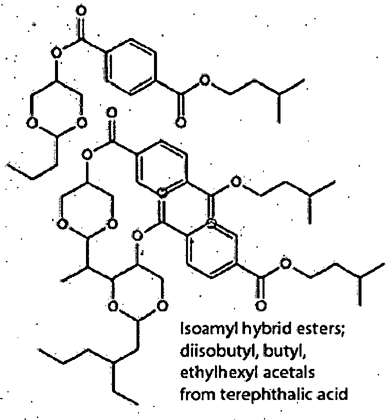

### Example 23: Synthesis of isoamyl hybrid esters; diisobutyl, butyl, ethylhexyl acetals from succinic acid:

In a glass reactor charge 220 grams of purified glycerol 95% equipped with condenser and water separator, and under continuous flow of nitrogen, add 0.7 grams of methane sulfonic acid, 230 grams of isobutyraldehyde or isobutyraldehyde (500grams in the case of ethylhexyl aldehyde, 120 grams of isoamyl alcohol and 100 grams of succinic acid).
Heat for 120 minutes to 180 °C and react until it reaches an acidity index lower than 3 mgkoh/gram of product. Apply vacuum of 100 mmHg to remove excess of reagents.
The isoamyl hybrid esters; isobutyl, butyl, ethylhexyl acetals fromsuccinic acid has purity over 90%.

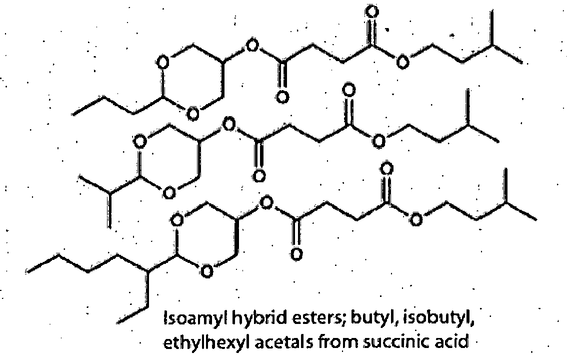

### Example 24: Synthesis of isoamyl hybrid esters; butyl, diisobutyl, ethylhexyl acetals from maleic anhydride.

In a glass reactor charge 210 grams of purified glycerol 95% equipped with condenser and water separator, and under continuous flow of nitrogen, add 0.7 grams of methane sulfonic acid, 230 grams of butyraldehyde or isobutyraldehyde (500grams in case of ethylhexaldehyde), 120 grams of isoamyl alcohol, 80 grams of maleic anhydride. Heat for 120 minutes to 180°C and react until it reaches an acidity index lower than 3 mgkoh/gram of product. Apply vacuum of 100 mmHg to remove excess of reagents:
The isoamyl hybrid esters; butyl, diisobutyl, ethylhexyl acetals of maleic acid has purity over 90%.

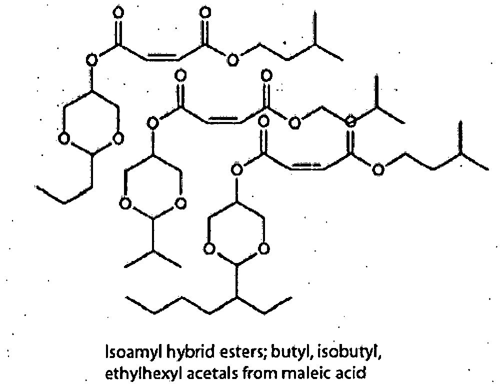

### Example 25: Synthesis of diisobutyl esters, butyl, ethylhexyl acetals and maleic anhydride:

In a glass reactor charge 220 grams of purified glycerol 95% equipped with condenser and water separator, and under continuous flow of nitrogen, add 0.7 grams of methane sulfonic acid, 230 grams of isobutyraldehyde or isobutyraldehyde (500grams in the case of ethylhexyl aldehyde, and 60 grams of maleic anhydride). Heat for 120 minutes to 180 °C and react until it reaches an acidity index lower than 3 mgkoh/gram of the product.
Apply vacuum of 100 mmHg to remove excess of reagents.
The isobutyl ester, butyl, ethylhexyl acetals of maleic anhydride has purity above 90%.

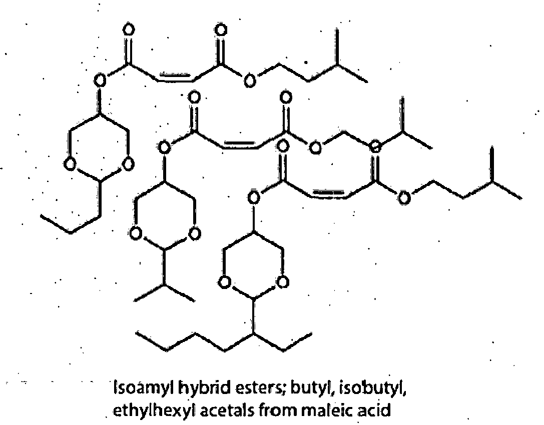

### Example 26: Synthesis of sulfosuccinate from hybrid esters:

In a glass reactor charge 210 grams of purified glycerol 95% equipped with condenser and water separator, and under continuous flow of nitrogen, add 0.7 grams of methane sulfonic acid, 230 grams of butyraldehyde or isobutiraidehyde (500g in case of ethylhexaldehyde), 180 grams of isoamyl alcohol, 160 grams of maleic anhydride. Heat for 120 minutes to 180 °C and react until it reaches an acidity index lower than 3 mgkoh/gram of the product. Apply vacuum of 100 mmHg to remove excess of reagents.
Approximately 200 grams of diester was subjected to sulfitation for producing the its respective sodium sulfosuccinate using 60 grams of sodium metabisulfite dissolved in 100 grams of water.
The sulfosuccinate of isoamyl esters; butyl, diisobutyl, ethylhexyl acetal esters obtained a 90% conversion in anionic active.

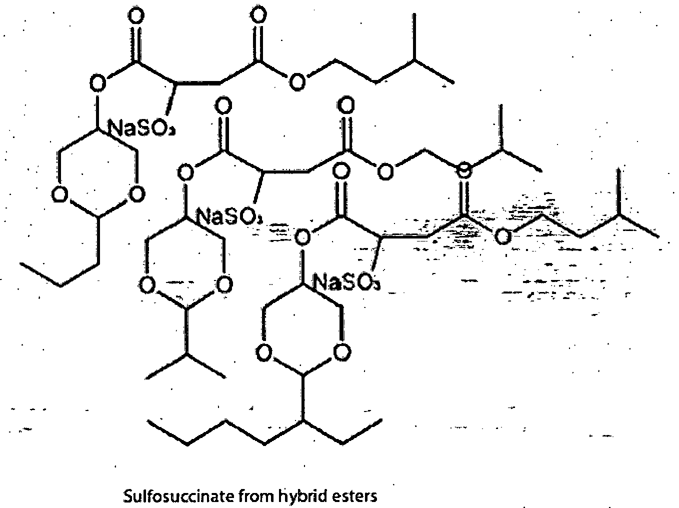

### Example 27: Synthesis of the sulfosuccinates of butyl, diisobutyl, ethylhexyl acetal esters:

In a glass reactor charge 210 grams of purified glycerol 95% equipped with condenser and water separator, and under continuous flow of nitrogen, add 0.7 grams of methane sulfonic acid, 230 grams of butyraldehyde or isobutyraldehyde (500 g in case of ethylhexyl aldehyde), 80 grams of maleic anhydride. Heat for 120 minutes to 180 °C and react until it reaches an acidity index lower than 3 mgkoh/ grams of the product. Apply vacuum of 100 mmHg to remove excess of reagents.
Approximately 200 grams of diester was subjected to sulfitation process for the production of its respective sodium sulfosuccinate using 60 grams of sodium metabisulfite dissolved in 100 grams of water.
The butyl sulfosuccinate, diisobutyl, ethylhexyl acetal esters obtained a conversion over 90% in anionic active.

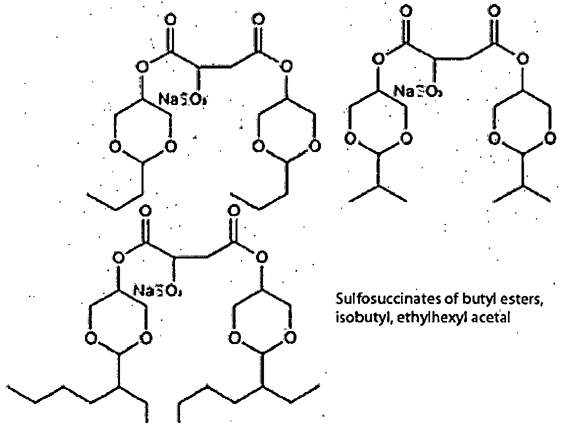

### Example 28: Synthesis of debutyl acetates; isobutyl, ethylhexyl acetals:

In a glass reactor charge 210 grams of distilled glycerin equipped with condenser and water separator, and under continuous flow of nitrogen, add 0.7 grams of methane sulfonic acid, 300 grams of butyraldehyde or isobutyraldehyde (550 g in case of ethylhexaldeide), heat for 120 minutes to 140 °C and react until completing the reaction or up to about 40 grams of water.
Apply vacuum of 100 mmHg to remove the excess of reagents.
In another reactor charge 150 grams of isobutyl acetal formed in the step described above, add 130g of acetic anhydride. React for 60 minutes to 110 °C, apply vacuum of 100 mmHg to remove the excess of reagents and the acetic acid formed.
Butyl acetates, isobutyl, ethylhexyl acetal with purity over 93% were formed.

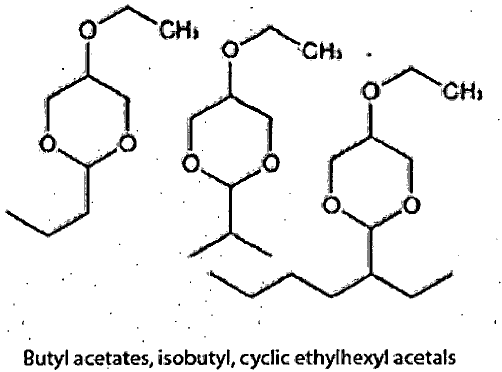

### Example 29: Synthesis of butyl abietate, isobutyl, ethylhexyl acetals:

In a glass reactor charge 210 grams of purified glycerol 95% equipped with condenser and water separator, and under continuous flow of nitrogen, add 0.7 grams of methane sulfonic acid, 230 grams of butyraldehyde or isobutyraldehyde (480 g in case of ethylhexyl aldehyde), 100 grams of abietic acid, known commercially as pitch. Heat for 120 minutes to 180 °C and react until it reaches an acidity index lower than 3 mgkoh/gram of product. Apply vacuum of 100 mmHg to remove excess of reagents.
Butyl abietate, isobutyl, ethylhexyl acetal with 95% purity were formed in this process.

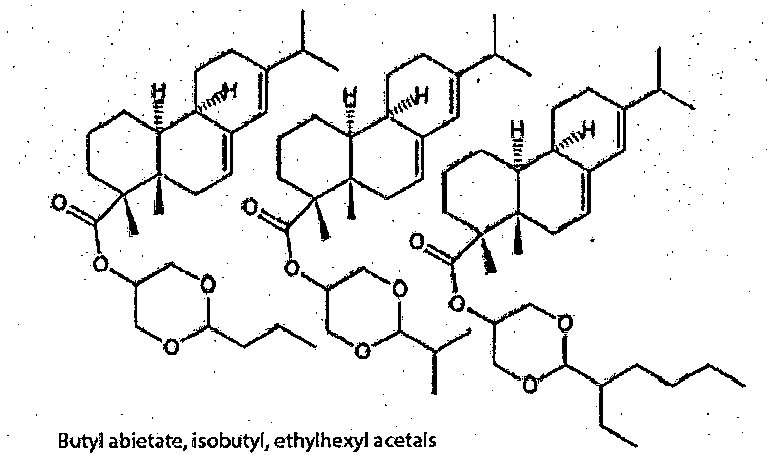

### Example 30: Synthesis of butyl monocarboxylic esters, isobutyl, ethylhexyl, acetals of fatty acids of such oil:

In a glass reactor charge 210 grams of purified glycerol 95% equipped with condenser and water separator, and under continuous flow of nitrogen, add 1.0 grams of methane sulfonic acid, 230 grams of butyraldehyde or isobutyraldehyde (in the case the ethylhexyl aldehyde load 500 grams), 150 grams of fatty acid of such oil commercially known as TOFA. Heat for 120 minutes to 180 °C and react until it reaches an acidity index lower than 3 mgkoh/gram of product. Apply vacuum of 100 mmHg to remove, excess of reagents.
Butyl ester, isobutyl, ethylhexyl acetals of TOFA fatty acids with purity over 93% were formed in this process.

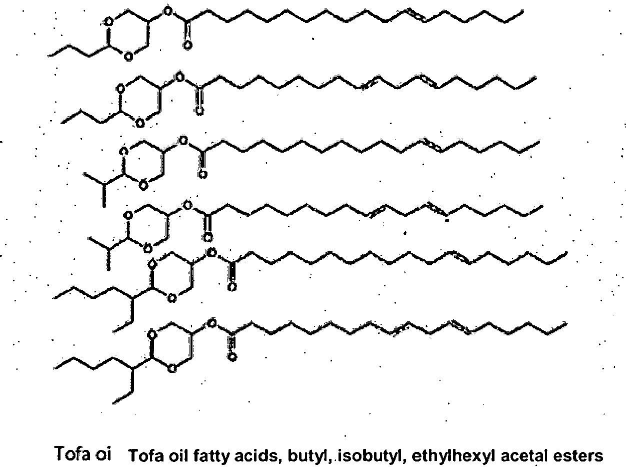

### Example 31: Synthesis of butyl acrylate, isobutyl, ethylhexyl acetals:

In a glass reactor charge 210 grams of purified glycerol 95% equipped with condenser and water separator, and under continuous flow of nitrogen, add 1.0 grams of methane sulfonic acid, 230 grams of butyraldehyde or isobutyraldehyde (in the case the ethylhexaldeide load 500 grams), 75 grams of acrylic acid, and 0.3 grams of hydroquinone. Heat for 120 minutes to 150°C and react until it reaches an acidity index lower than 3 mgkoh/gram of product. Apply vacuum of 100 mmHg to remove excess of reagents.
Butyl acrylate, isobutyl, ethylhexyl acetals with purity over 93% were formed in this process.

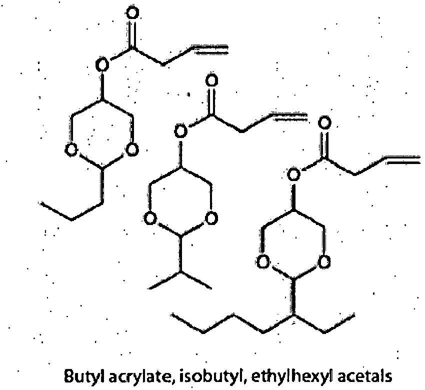

### Example 32 : Synthesis of the epoxidized unsaturated fatty acids from soybean oil, linseed, sunflower, canola, corn, peanut, such oil:

In a glass reactor charge 210 grams of distilled glycerol equipped with condenser and water separator, and under continuous flow of nitrogen, add 1.0 grams of 85% phosphoric acid. Heat the reaction mass to 110°C and add into the reaction mass 224 grams of isobutyraldehyde in 60 minutes of addition. Keep reacting for 180 minutes until all the water from the reaction is separated.
Add 480 grams of unsaturated soybean fatty acid (which may be still linseed, sunflower, canola, corn, peanut, such oil) and 0.5 grams of dibutyltin dilaurate. Heat for 120 minutes to 180°C and react until it reaches an acidity index less than 2.5 mgkoh/gram of product. Apply a vacuum of 100 mmHg to remove the excess of reagents.
The acetal ester from the unsaturated fatty acid was epoxidized using the conventional technique with hydrogen peroxide and formic acid (performic acid in situ) getting its respective acetal ester from the epoxidized fatty acid. The yield of epoxy was 80 % compared to the number of unsaturations.
The figure below shows the structure of the products obtained with oleic and linoleic chain.

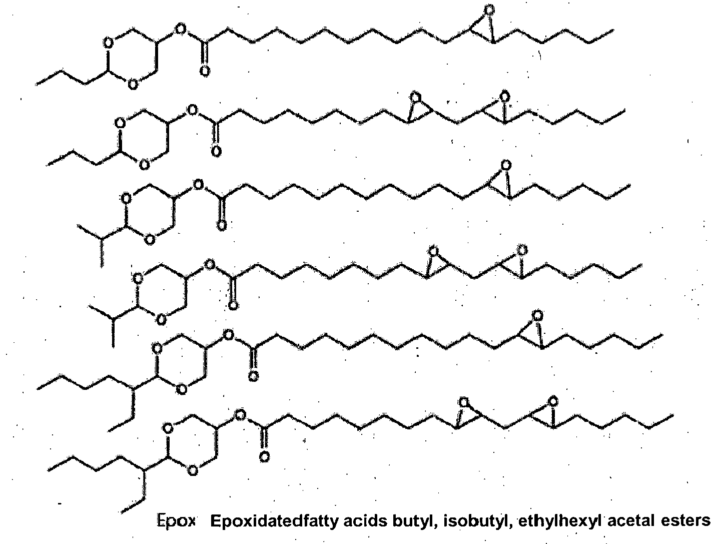

## Claims

1. **"ACETAL ESTERS PRODUCED FROM PURIFIED GLYCERIN FOR USAGE AND APPLICATIONS AS EMOLLIENTS, LUBRICANTS, PLASTICIZERS, SOLVENTS, COALESCENTS, HUMECTANTS, POLYMERIZATION MONOMERS, ADDITIVES TO BIOFUELS",** more particularly it concerns mono-and diesters of acetals ***characterized by** :*
- being produced from glycerine, organic aldehydes, and carboxylic or dicarboxylic acids such as humectants, solvents, additives, lubricants, softeners, plasticizers, surfactants, dispersants;
- products produced from the techniques described may contain natural antioxidants from purified glycerin whose process prevents distillation and preserves the presence of tocopherols and other active ingredients such assterols and squalene;
- the glycerin used may be technical distilled glycerin, USP glycerin, blond glycerin, and preferably purified glycerin;
- organic aldehydes can be propinaldehyde, isoamialdehyde, nonaldehyde, decanaldehyde, isotridecilaldehyde, furfuraldehyde, benzaldehyde, and preferably butyraldehyde, isobutyraldehyde and ethylhexaldehyde;
- Carboxylic acids are acetic acid, propionic acid, butyric acid, hexoic acid,capric acid, caprylic acid, 2-ethylhexanoic acid, nonanoic acid, decanoic acid, lauric acid, palmitic acid, myristic acid, stearic acid, oleic acid, linoleic, linolenic acid, ricinoleic acid,arachidonic acid, lactic acid, citric acid, benzoic acid, acrylic acid, methacrylic acid and their respective methyl and ethyl esters;
- The dicarboxylic acids may be adipic acid, orthophthalic acid, maleic acid, succinic acid, terephthalic acid, oxalic acid, maleic acid, dimerized fatty acids and their respective methyl and ethyl diesters;
- The alcohols used for the synthesis of hybrid ester may be the following: methyl, ethyl, propyl, butyl, isobutyl, amyl, isoamyl, hexanol, octanol, decanol, dodecanol, cetostearyl alcohols, guebert, oleyl, preferably isoamyl alcohol from natural origin;
- The synthesis techniques can be:
- The production of acetal with subsequent esterification or transesterification reaction, or preferably with *in situ* formation of the acetal, or acetal and ester are being made in a single step; molar ratios between glycerol: aldehyde: carboxyl group in the range of 10:20:1 to 3:6:1 preferablyl.5:2.0:1;
- Reaction temperature in the range of 80°C to 250°C, preferably 140°C to 180°C;
- Catalysts are organometallic acids and acids which may be sulfuric acid, phosphoric acid, methanesulfonic acid, para toluene sulfonic acid, benzene sulfonic acid , trifluorrrietanosulfonic acid, hydrochloric acid, nitric acid, tin oxalate, tin oxalate , and all the organic derivatives-of tin, preferably methane sulfonic acid, para toluene sulfonic acid, tin oxalate, trifluoromethanesulfonic acid;
- The amount of catalyst is 100 parts per million to 100,000 parts per million, preferably from 500 parts per million to 5,000 parts per million.

2. **"ACETAL ESTERS"** according to claim 1 and according to example 1, *characterized* as butyl laurate, isobutyl, ethylhexyl acetal, due to the presence of lauric fat chain, cyclic ether group, the ester function are components of lubricant formulations for industrial usage such as products such to be used as solvents and as a component in formulations for cutting fluids for metal laminations such as aluminum, refrigeration lubricants, and lubricants for drilling oil wells.

3. **"ACETAL ESTERS",** according to claim 1 *characterized*as butyl laurate, isobutyl, ethylhexyl acetal, due to lauric fat chain, cyclic ether group , ester function are components of cosmetic formulations acting as a solvent and diluent for active ingredients as sunscreens and giving spreadability on the skin and with the presence of tocopherals in the components whichact as antioxidants to be employed to preserve the skin against aging.

4. **"ACETAL ESTERS",** according to claim 1 *characterized*as capric and caprylic esters which act as donor agent of oiliness in cosmetic formulations; the synthesis of ester using purified glycerin containing natural tocopherols in its composition have additional functions as antioxidants and retardant agent against aging and as plasticizer or component for nail polish.

5. **"ACETAL ESTERS",** according to claim 1 *characterized*by capric and caprylic esters are components of formulations such as plasticizers for use as synthetic or natural rubber, cellulose, nitrocellulose, acrylic resins , because of their high compatibility with polar solvents such as polymers or herbicides and insecticides formulations due to the high functionality.

6. **"ACETAL ESTERS**"of fatty acids of carbon chain C12 and C18, its mixtures or individually pure according to Claim 1, ***characterized* by** the presence of lauric fat chain, cyclic ether group, the ester function of this product act in cosmetic formulations with the function to reset the oiliness and spreadability insurfaces as the skin, and yet, due to the presence of tocopherols which act as antioxidants to preserve the skin against aging.

7. **"ACETAL ESTERS"**from the fatty acid of carbon chain C12 to C18, its mixtures or individually pure according to Claim 1, ***characterized* by** the presence of lauric fat chain, cyclic ether group, the ester function of this product act in the formulations in cutting fluids and metal laminates such as aluminum, and also due to acetal esters of fatty acids of the C12 to C18 carbon chain from of glycerin reaction, ethylhexyl aldehyde, C12 to C18 fatty acidsact as components of industrial lubricants for metallaminates, cutting fluids, leather industry for processing textiles such as threads and fabricsas well as in biofuel industry for usage as additives in combustion processes of biofuels and to reduce and adjust the point of cold plugging of biofuels made from vegetable oils and animal fats.

8. **"ACETAL ESTERS"**from 2-ethylhexanoic acid from the reaction of glycerin, butyraldehyde or isobutyraldehyde or ethylhexyl aldehyde, and 2-ethylhexanoic according to Claim 1, **characterized by** acting as components of industrial lubricants for refrigeration, mental laminates, cutting fluids, leather industry and lubricants for the processing of textile and fabrics.

9. **"ACETAL ESTERS"** from the fatty acid carbon of unsaturated chain such as the fatty acids from soybean, or mixtures thereof and individually pure according to claim 1, **characterized by** thepresence of oleic and linoleicfat cliain, cyclic ether group, from the ester function to compose oiliness and spreadability on surfaces such as the skin and the presence of tocopherols acting asantioxidants preserving the skin against aging applicable in cosmetic formulations,

10. **"ACETAL ESTERS**" from the unsaturated carbon chain such as fatty acids from soybean, its mixtures or individually pure according to Claim 1, **characterized by** the presence of oleic and linoleic chain, cyclic ether group, from the ester function to compose oiliness to be used as components in formulations for cutting fluids and metal laminates such as aluminum and as solvents in herbicides and insecticides formulations to replace mineral oils, fatty methyl esters, ethyl lactate and organic amides, and in the biofuel industry as stated.

11. **"ACETAL ESTERS"** from the fatty acids of unsaturated carbon chain as the fatty acids from soybean, its mixtures thereof and individually pure and according to Claim 1, **characterized by** the presence of oleic and linoleic chain, cyclic ether group, from the ester function to be used as additives and coalescing agents in formulations of inks based on acrylic resins.

12. **"ACETAL DIESTERS"** from orthophthalic anhydride derived from the glycerin reaction, butyraldehyde or isobutyraldehyde or ethylhexyl aldehyde, and the phthalic anhydride according to claim 1, characterized to be acting as plasticizers and solvents for polar polymers such as polyvinyl chloride, nitrocellulose, acrylics due to its high polarity, low volatility, chemical stability and the presence of tocopherols that act as antioxidants in polymeric systems as described above, including low exudation properties, high transparency, color stability and high flexibility.

13. **"ACETAL DIESTERS"** from the adipic acid derived from the glycerin reaction, butyraldehyde or isobutyraldehyde or ethylhexyl aldehyde with adipic acid, according to claim 1, characterized to be acting as plasticizers named free of phthalates for polar polymers such as polyvinyl chloride, nitrocellulose, acrylics due to its high polarity, low volatility, chemical stability and the presence of tocopherols that act as antioxidants in polymeric systems as described above including low exudation properties, high transparency, color stability and high flexibility.

14. **"ACETAL DIESTERS"** from the adipic acid from the glycerin reaction, butyraldehyde or isobutyraldehyde or ethylhexyl aldehyde with adipic acid, according to claim 1, characterized to be acting as plasticizers for nail polish.

15. **"ACETAL DIESTERS"** from the terephthalic acid from the glycerin reaction, butyraldehyde or isobutyraldehyde or ethylhexyl aldehyde with terephthalic acid according to claim 1, characterized to be acting as plasticizers named free of phthalates for polar polymers such as polyvinyl chloride, nitrocellulose, acrylics due to its high polarity, low volatility, chemical stability and the presence of tocopherols that act as antioxidants in polymeric systems as described above including low exudation properties, high transparency, color stability and high flexibility.

16. **"ACETAL DIESTERS"** from the succinic acid from the glycerin reaction, butyraldehyde or isobutyraldehyde or ethylhexyl aldehyde with succinic acid according to claim 1, characterized to be acting as plasticizers named free of phthalates for polar polymers such as polyvinyl chloride, nitrocellulose, acrylics due to its high polarity, low volatility, chemical stability and the presence of tocopherols that act as antioxidants in polymeric systems as described above including low exudation properties, high transparency, color stability and high flexibility.

17. **"ACETAL DIESTERS"** from adipic acid acetals from the glycerin reaction, butyraldehyde or isobutyraldehyde or ethylhexyl aldehyde, isoamyl alcohol with adipic acid according to claim 1, characterized to be acting as plasticizers named free of phthalates for polar polymers such as polyvinyl chloride, nitrocellulose, acrylics due to its high polarity, low volatility, chemical stability and the presence of tocopherols that act as antioxidants in polymeric systems as described above including low exudation properties, high transparency, color stability and high flexibility.

18. **"ACETAL DIESTERS"** from adipic acid f acetals from the glycerin reaction, butyraldehyde or isobutyraldehyde or ethylhexyl aldehyde, isoamyl alcohol with adipic acid according to claim 1 and due to the fact of having as component the isoamyl alcohol from the etanol production from sugar cane, characterizedto be employed in formulations as green solvents.

19. **"ACETAL HYBRID DIESTERS"** from orthophthalic anhydride or terephthalic acid or sulfosuccinic acid from the glycerin reaction, butyraldehyde or isobutyraldehyde or ethylhexyl aldehyde, isoamyl alcohol with orthophthalic anhydride or terephthalic acid or succinic acid and according to claim 1, **characterized by** acting as plasticizers act with a great power of solvency and viscosity regulator named free of phthalates for polar polymers such as polyvinyl chloride, nitrocellulose, acrylics due to its high polarity, low volatility, chemical stability and the presence of tocopherols that act as antioxidants in polymeric systems as described above including low exudation properties, high transparency, color stability and high flexibility.

20. **"ACETAL HYBRID DIESTERS"** from orthophthalic anhydride or terephthalic acid or sulfosuccinic acid from the glycerin reaction, butyraldehyde or isobutyraldehyde or ethylhexyl aldehyde, isoamyl alcohol with orthophthalic anhydride or succinic acid or terephthalic acid according to claim 1 and for the fact of having as component the isoamyl alcohol derived from the ethanol production from sugar cane, characterized to be employed in formulations as green solvents.

21. **"HYBRID DIESTERS"** from maleic anhydride used in the production of sulphosuccinates according to claim 1, characterized to be used as humectants in water based inks, agricultural formulations, surfactants and dispersants for inks.

22. **"HYBRID DIESTERS"** from maleic anhydride used in the production of sulfosuccinates as an alternative and according to claim 1, characterized to be used as dewatering agents in metal laminates as aluminum.

23. **"HYBRID DIESTERS"** from maleic anhydride used in the production of sulfosuccinates as an alternative in accordance with claim 1, characterized to be used as monomers or as comonomers for the production of polymers based on vinyl polyacetate in adhesives and inks.

24. **"ACETAL ESTER ACETATES"** from glycerin reaction, butyraldehyde or isobutyraldehyde or ethylhexyl aldehyde and acetic anhydride or acetic acid in accordance with claim 1, characterized to be used as solvents and additives in cleaning product formulations as detergents, paint solvents, plasticizers for cellulose, nitrocellulose, natural or synthetic rubber.

25. **"ACETAL ABIETATES"** from glycerin reaction, butyraldehyde or isobutyraldehyde, ethylhexyl aldehyde and abietic acid according to claim 1, characterizedto be used as components in the manufacture of thermoplastic resins for the production of industrial inks and adhesives.

26. **"ACETAL ESTERS"** from fatty acids of unsaturated carbon chain such as the fatty acids o such oil, its mixtures or individually pure according to claim 1, characterized to be used as component of lubricant formulations for industrial use and cutting fluid; or even as lubricants for metal laminations as aluminum.

27. **"ACETAL ESTERS"** from fatty acids of unsaturated carbon chain such as the fatty acids of such oil, its mixtures or individually pure according to claim 1, characterized to be used as solvents in herbicide and insecticide formulations replacing mineral oils, fatty acid methyl esters, ethyl lactate and organic amides.

28. "**ACETAL ESTERS**" from fatty acids of carbon chain such as the fatty acids of such oil and abietic acids, its mixtures or individually pure according to claim 1, characterized to be employed as component of lubricant formulations for industrial use such as component in formulations for cutting fluids and for metal laminates such as aluminum.

29. **"ACETAL ESTERS"** from the fatty acids of chain carbonic as fatty acids of such oil and abietic acids, its mixtures or individually pure according to claim 1, characterized to be employed as component of cosmetic formulations, since the presence of lauric fatty chain, cyclic ether group, the ester function of this product includes properties of oiliness and spreadability on surfaces such as the skin and, furthermore, the presence of tocopherols that act as antioxidants act also as protection component to the skin aging.

30. **"ACETAL ESTERS**" from the fatty acids of chain carbonic as fatty acids of such oil and abietic acids, its mixtures or individually pure according to claim 1, characterized to be employed as solvents in herbicides and insecticide formulations replacing mineral oils, fatty acid methyl esters, ethyl lactate and organic amides.

31. **"BUTYL ACRYLATES, ISOBUTYL, ETHYLHEXYL ACETALS"** according to claim 1, characterized to be employed as polymerization monomers for the production of inks and varnishes replacing the conventional monomers as ethyl acrylates, butyl acrylate and ethylhexyl acrylates.

32. **"ACETAL ESTERS OF EPOXIDIZED FATTY ACIDS"** according to claim 1, characterized to act as plasticizers since the applications of these products belong to the cosmetic field as enamel plasticizers; in industry as plasticizers for compositions of polyvinyl chloride (PVC) to produce plastisols used in the automotive industry, flexible PVC films , PVC plastic parts for toys, PVC hoses, PVC shoes and their blends; in the paint industry as plasticizers for nitrocellulose resins, polyurethane-based resins; in the adhesive industry as plasticizers for acrylic resins, vinyl polyacetates; polyamide resins; in the agricultural industry as stabilizingagents of chlorinated substances; preferably this product group applies in the industry of PVC-based flexible plastics and styrene and butadiene-based rubbers.

33. **"ACETALS ESTERS PRODUCED FROM PURIFIED GLYCERIN FOR USE AND APPLICATIONS AS EMOLLIENTS, LUBRICANTS, PLASTICIZERS, SOLVENTS, COALESCENTS, HUMECTANTS, POLYMERIZATION MONOMERS, ADDITIVES TO BIOFUEL",** characterized because the application of these products belong to the cosmetic field as enamel plasticizers; in industry as plasticizers for compositions of polyvinyl chloride (PVC) to produce plastisols used in the automotive industry, flexible PVC films, PVC plastic parts for toys, PVC hoses, PVC shoes and their blends; in the paint industry as plasticizers for nitrocellulose resins, polyurethane -based resins; in the adhesive industry as plasticizers for acrylic resins, vinyl polyacetates; polyamide resins; in the agricultural industry as stabilising agents of chlorinated substances;preferably this product group applies in the industry of PVC-based flexible plastics and styrene and butadiene-based rubbers.
